(19)

**Europäisches Patentamt**
**European Patent Office**
**Office européen des brevets**

(11) **EP 4 229 390 B1**

(12) **EUROPEAN PATENT SPECIFICATION**

(45) Date of publication and mention
of the grant of the patent:
**11.06.2025 Bulletin 2025/24**

(21) Application number: **21794136.8**

(22) Date of filing: **14.10.2021**

(51) International Patent Classification (IPC):
$G01N\ 1/22$ (2006.01)   $A61B\ 5/00$ (2006.01)
$A61B\ 5/08$ (2006.01)   $A61B\ 5/09$ (2006.01)
$A61B\ 5/091$ (2006.01)   $A61B\ 5/097$ (2006.01)

(52) Cooperative Patent Classification (CPC):
**G01N 1/2208; A61B 5/082; A61B 5/09;**
**A61B 5/091; A61B 5/097;** G01N 2001/2244

(86) International application number:
**PCT/EP2021/078512**

(87) International publication number:
**WO 2022/079197 (21.04.2022 Gazette 2022/16)**

(54) **A COLLECTING DEVICE AND A METHOD FOR COLLECTION OF AIRBORNE PARTICLES FROM A FLOW OF AIR**

SAMMELVORRICHTUNG UND VERFAHREN ZUM SAMMELN VON LUFTPARTIKELN AUS EINEM LUFTSTROM

DISPOSITIF DE COLLECTE ET PROCÉDÉ DE COLLECTE DE PARTICULES EN SUSPENSION DANS L'AIR À PARTIR D'UN FLUX D'AIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2020 EP 20201787**
**26.04.2021 EP 21170431**

(43) Date of publication of application:
**23.08.2023 Bulletin 2023/34**

(73) Proprietor: **Imec VZW**
**3001 Leuven (BE)**

(72) Inventors:
• **TAHER, Ahmed**
**3001 Leuven (BE)**
• **JONES, Benjamin**
**3001 Leuven (BE)**
• **PEUMANS, Peter**
**3001 Leuven (BE)**

(74) Representative: **AWA Sweden AB**
**Box 5117**
**200 71 Malmö (SE)**

(56) References cited:
US-A1- 2004 118 222     US-A1- 2005 058 575
US-A1- 2005 279 181     US-A1- 2016 223 435
US-A1- 2020 268 280     US-A1- 2020 278 275

## Description

Cross-reference to related applications

This application claims the benefit of European Patent Application No. EP20201787.7 filed 14 October 2020 and European Patent Application No. EP2/1170431.7 filed 26 April 2021.

Technical field

The present inventive concept relates to a collecting device and a method for collection of airborne particles from a flow of air.

Background

Efficient collection of airborne particles exhaled by a human to facilitate analysis is of high interest in various applications. For instance, enabling detection of particular substances in a human breath may provide the possibility of screening for infectious diseases, such as influenza and severe acute respiratory syndrome coronavirus 2 (SARS-CoV-2). Thus, collection of exhaled airborne particles in an efficient and inexpensive way may allow screening to be performed frequently, allow quick identification of persons carrying disease, and consequently reducing spreading of the disease. Several diseases, such as influenza and SARS-CoV-2, spread among humans through droplets and aerosols produced during breathing, blowing, talking, coughing, and sneezing. Thus, it would be of interest to provide a collecting device allowing efficient capture of airborne particles exhaled by a human being and facilitating analysis of substances within the collected particles so as to identify whether the person carries a disease or not. US 2016/0223435 A1 discloses an airborne substance sensing device comprising a cartridge having an introduction plate in which micropores are formed through which a gas including an airborne substance can pass, a transparent collection plate that is disposed so as to oppose the introduction plate and on which the airborne substance can be made to collide and be collected through the collision of the gas having passed through the micropores.

US 2020/0278275 A1 discloses a kit of parts for assembly into an impactor for aerosol component collection. An impactor component is receivable into the assembly such that, when a sealing component is in a sample collection configuration and an aerosol sample is being received at an aerosol inlet of a housing of the kit, in use, an aerosol flow path of the aerosol sample is directed onto an impaction surface of the impactor component to promote aerosol deposition thereon.

Summary

An objective of the present inventive concept is to provide a collecting device allowing simple and efficient collection of airborne particles exhaled by a human being.

This and other objectives of the present inventive concept are at least partly met by the invention as defined in the independent claims. Preferred embodiments are set out in the dependent claims.

According to a first aspect, there is provided a collecting device for collection of airborne particles from a flow of air, said collecting device comprising: a first layer and a second layer, wherein the first layer and the second layer are arranged to be spaced apart for forming a particle collection chamber between the first and the second layer, wherein the first layer comprises a plurality of inlets configured to extend through the first layer for transporting the flow of air therethrough into the particle collection chamber; wherein ends of the inlets are configured to face a first surface of the second layer for capturing airborne particles in the flow of air entering the particle collection chamber through the ends of the inlets by impaction of airborne particles on the first surface of the second layer; wherein the second layer comprises a plurality of outlets configured to extend through the second layer for transporting the flow of air therethrough out of the particle collection chamber; wherein the inlets and outlets are staggered such that the center axes of the inlets are displaced from the center axes of the outlets; wherein the collecting device is configured such that the flow of air experiences a pressure drop when passing the collecting device, the pressure drop being lower than 3 kPa at a flow rate of 0.5 liters per second.

Thanks to the collecting device, an efficient capturing of airborne particles may be provided in the particle collection chamber. The use of a plurality of inlets may ensure that a high capturing efficiency of airborne particles is achieved in the particle collection chamber, as capturing of airborne particles occur in multiple positions of the particle collection chamber (associated with respective inlets). Further, thanks to the inlets and the outlets being staggered, the inlets are arranged directly above an unbroken part of the first surface of the second layer wherein capturing of airborne particles occur. Thus, airborne particles may be captured at the unbroken part of the first surface, while the flow of air through the inlets changes direction to follow the first surface and then escape the particle collection chamber through the outlets.

As used herein, capturing of airborne particles by "impaction" should be construed as particles being removed from the flow of air by forcing the flow of air to change direction. Thanks to the flow of air being forced to change direction, momentum of particles having a certain size will cause the particles not to follow the flow of air in its change of direction and instead the particles will be captured on a collection surface. The capturing of airborne particles may involve capturing of aerosols but may also or alternatively involve capturing of larger droplets in the flow of air.

Further, thanks to the collecting device comprising a plurality of inlets and a plurality of outlets, the collecting device facilitates breathing through the collecting device without requiring a high supply pressure to be provided by the person breathing through the collecting device. Thus, the flow of air may be provided by being exhaled by a human being. The flow of air exhaled by the human being may follow a path through the collecting device such that substantial changes in dimensions of a cross-section of the path are avoided and, hence, large pressure drops are avoided. In particular, the use of a plurality of inlets and outlets enables the flow of air to experience a combined cross-section of the respective inlets and outlets that is relatively large such that a large pressure drop may be avoided even though individual inlets and outlets may have small cross-sections. Further, the small cross-sections facilitate particle collection at low flow rates of the flow of air, such as in the order of 0.5 liters per second.

The collecting device allows a human being to breath or blow into the collecting device such that particles comprised in the exhaled air can be conveniently collected without requiring difficult procedures for the person. Compared to sample collection through a nasal or throat swab, or saliva collection, the collecting device provides a particle collection with much less discomfort for the user and may remove the need for a trained professional to be involved in the sample collection. Thanks to the collecting device being designed such that a pressure drop when the flow of air passes the collecting device is lower than 3 kPa at a flow rate of 0.5 liters per second, the required pressure supplied by the human being in exhaled air can be easily achieved by the human being without causing discomfort.

The flow of air may be received by the collecting device by the human being blowing directly into an apparatus holding the collecting device, such as through a mouthpiece of the apparatus. Hence, the flow of air may be provided directly from exhalation by the human being. However, it should be realized that the flow of air may alternatively be received indirectly from the human being, e.g. by the human being exhaling into a bag and by the flow of air being provided by the bag towards the collecting device. Further, the flow of air need not necessarily originate from a human being. Rather, the flow of air may be based on exhale from another living being or may be based on air being collected in any environment and being provided into an apparatus holding the collecting device. A required pressure for passing the flow of air through the collecting device need thus not be provided by the exhale of a human being but may be provided by any component causing a flow of air.

In the following, the flow of air may be referred to as originating from exhale of a human being, but it should be realized that the flow of air may alternatively be provided in other manners.

As used herein, the term "flow of air experiences a pressure drop when passing the collecting device" should be construed as the pressure drop experienced along an entire path from an inlet of the collecting device (defined by open ends of the inlets allowing the flow of air to enter the collecting device) to an outlet of the collecting device (defined by open ends of the outlets allowing the flow of air to leave the collecting device). Thus, the experienced pressure drop may occur in the inlets, in the particle collection chamber and in the outlets or in a combination thereof.

It should be realized that the pressure drop depends on the flow rate through the collecting device. The flow rate of the flow of air through the collecting device based on air exhaled by the human being will depend on the exhalation flow rate provided by the human being. Thus, the actual pressure drop that will occur in the collecting device will depend on the exhalation flow rate that is provided. However, the collecting device according to the first aspect is designed such that, when a flow rate of 0.5 liters per second is received through the collecting device, the pressure drop is lower than 3 kPa.

The collecting device may facilitate an easy and intuitive way of collecting samples through self-test by persons, thereby promoting more frequent testing of people. In this regard, the use of the collecting device for screening of people may allow people to be screened several times per week, such as every day, such that viral load peaks may be identified. If testing is done only once a week, the viral load peak may be missed such that the testing fails to detect persons having a stage of a disease in which the person is highly infectious. Hence, very frequent testing may be required in order to identify highly infectious people and prevent spreading of a disease.

The particle collection chamber may be adapted for allowing reactions to take place therein and/or for allowing measurements to be performed in relation to the collected particles in the particle collection chamber. Thus, analysis of the collected particles may be performed in the particle collection chamber.

The particle collection chamber may thus be utilized also for analysis of collected particles. The analysis of the collected particles may be performed through a light-based measurement, such as by illuminating the collected particles and/or substance(s) extracted from the collected particles and detecting light after interaction with the collected particles and/or by detecting light based on some other type of stimulation, such as inducing a chemical reaction or providing thermal or electrical energy for causing output of light. Light interaction may include absorption, transmission, scattering, and fluorescence.

Optical access to the particle collection chamber may be provided by the first and/or the second layer being transparent to wavelengths of light being used in the light-based measurement. Additionally or alternatively, optical access may be provided through the inlets and/or the outlets defining a space (which may possibly be filled by a liquid) through which optical access to the particle collection chamber is provided.

According to an embodiment, the airborne particles may be borne e.g. by aerosols and/or by droplets in the air exhaled by a person. The airborne particles may further comprise substance(s) of interest, such that analysis of the collected particles may include the particles being subject to reactions for exposing the substance(s) of interest to enable detection of substance(s) of interest in a measurement.

Thanks to the collecting device being further configured to allow reactions and measurements to be performed while the collected particles are arranged in the particle collection chamber in which the collected particles are collected, the collecting device facilitates a fast analysis of a sample being made.

The first layer and the second layer may be parallel and define planar surfaces on opposite sides of the particle collection chamber. It should further be realized that the particle collection chamber may further be defined by side walls surrounding the particle collection chamber. The side walls may also provide a spacer for defining a distance between the first layer and the second layer.

The inlets and the outlets may extend from the particle collection chamber to an external environment outside the collecting device. Thus, the inlets may extend through at least the first layer and possibly further layers between the particle collection chamber and the external environment. Similarly, the outlets may extend through at least the second layer and possibly further layers between the particle collection chamber and the external environment.

The inlets may further be defined by walls extending from the first layer into the particle collection chamber and towards the second layer. Thus, the ends of the inlets facing the first surface of the second layer need not be flush with the first layer. Rather, the ends of the inlets may be arranged close to the second layer, which may facilitate capturing of particles, while the distance between the first and second layers may be larger than the distance between the ends of the inlets and the second layer to allow a larger volume of the particle collection chamber.

The inlets and the outlets being staggered implies that the inlets and the outlets are not aligned. The inlets and the outlets may preferably be parallel. Since the inlets and the outlets are staggered, positions of the center axes of the inlets in the particle collection chamber (e.g. at a plane through the center of the particle collection chamber between the first and second layers) are displaced from positions of the center axes of the outlets in the particle collection chamber (e.g. at the plane through the center of the particle collection chamber between the first and second layers).

Thanks to the center axes being displaced, the flow of air will need to change direction through the collecting device when passing from the inlets to the outlets. However, it should be realized that there may be an overlap between edges of the inlet and outlets, such that a projection of an end of the inlet facing the first surface of the second layer would overlap with an end of the outlet in the first surface of the second layer. It may be preferred that there is no overlap between edges of the inlet and outlets, since this would be associated with a relatively large change of direction of the flow of air, which may be advantageous in providing an efficient capturing of particles.

At least the first and second layers of the collecting device may be formed from a semiconductor or semiconductor-based material, such as silicon or silicon dioxide. This may facilitate manufacturing of the collecting device, since small dimensions of the collecting device may be advantageously provided by semiconductor manufacturing processes.

According to an embodiment, a volume of the particle collection chamber is smaller than 30 µl, such as smaller than 20 µl.

A small volume of the particle collection chamber facilitates performing fast analysis of a sample in the particle collection chamber.

The particle collection chamber may typically be filled with a reagent after collection of particles in the particle collection chamber. Then, the sample in the particle collection chamber may need to be heated and/or cooled, possibly in numerous iterations, in order to prepare the sample for analysis. Having a small volume of the particle collection chamber implies that steps of heating and/or cooling the sample in the particle collection chamber may be quickly performed.

However, the volume of the particle collection chamber may also affect the pressure drop that the flow of air experiences when passing the collecting device. A small volume of the particle collection chamber means that dimensions of the particle collection chamber need to be small such that pressure drop of the flow of air will be relatively large. Therefore, there may be a trade-off between the small size of the particle collection chamber for facilitating fast analysis and a pressure drop experienced by the flow of air passing through the collecting device. In some embodiments, a volume of the particle collection chamber may therefore need to be sufficiently large to avoid too large pressure drops experienced by the flow of air, such as the volume of the particle collection chamber being larger than 7 µl, such as larger than 10 µl. Thus, in some embodiments, the volume of the particle collection chamber is in a range of 7-30 µl, such as in a range of 10-20 µl.

According to an embodiment, the collecting device is configured such the pressure drop experienced by the flow of air when passing the collecting device is lower than 1.5 kPa at a flow rate of 0.5 liters per second.

Thanks to the collecting device being designed such that a pressure drop when the flow of air passes the collecting device is lower than 1.5 kPa at a flow rate of 0.5 liters per second, the required pressure supplied by the human being in exhaled air can be easily achieved by the human being without causing discomfort. A pressure drop as low as 1.5 kPa at a flow rate of 0.5 liters per second implies that adult human beings should be able to easily blow through the collecting device even if suffering from a disease affecting the lungs, such that a shortage of breath is experienced.

According to an embodiment, the collecting device is configured to provide a collection efficiency of at least 50% for particles having a diameter larger than 300 nm when the collecting device receives a flow of air with a flow rate of 0.5 liters per second.

This implies that particles of a diameter as small as 300 nm will be efficiently captured by the collecting device. The collection efficiency is higher for larger particles, such that particles having a diameter larger than 300 nm will also be efficiently captured. However, the collection efficiency drops fast towards smaller particles, such that a collecting device having 50% collection efficiency for particles of a diameter of 300 nm will have a low collection efficiency for smaller particles.

Thanks to efficiently collecting particles of a diameter of at least 300 nm, the collecting device is configured to efficiently collect particles that may carry infectious diseases. Hence, the collecting device could be used for efficiently collecting aerosols and/or larger droplets which may carry virus particles, such as severe acute respiratory syndrome coronavirus 2 (SARS-CoV-2).

The desired size of particles to be collected with at least 50% collection efficiency may be used as a design parameter of the collecting device. In addition or alternatively, the pressure drop to be experienced by the flow of air passing through the collecting device and/or the volume of the particle collection chamber may be used as design parameters.

Using such design parameter(s), dimensions and properties of other features of the collecting device may be determined. For instance, dimensions and number of inlets, dimension and number of outlets, arrangement of the inlets in relation to the outlets, dimensions of the particle collection chamber that may affect the pressure drop to be experienced by the flow of air passing through the collecting device, the volume of the particle collection chamber, and the size of particles that will be collected with at least 50% collection efficiency may be used as design parameters.

According to an embodiment, a smallest dimension of a cross-section of the inlets is in a range of 20 - 300 $\mu$m, such as in a range of 100 - 200 $\mu$m.

The size and shape of the inlets may be chosen in relation to at least a desired efficiency of collection of particles, a desired size of particles to be collected, and a pressure drop to be experienced by the flow of air when passing through collecting device.

In order to efficiently collect particles having a small diameter, the smallest dimension of the cross-section of the inlets needs to be small. Hence, in order to enable capturing smaller particles, the smallest dimension of the cross-section of the inlets should be decreased.

In order to ensure that the pressure drop to be experienced by the flow of air when passing through the collecting device is small, the smallest dimension of the cross-section of the inlets may need to be large (and not differ substantially from dimensions of other cross-sections passed by the flow of air in a path through the collecting device). However, it should be realized that the pressure drop need not necessarily be larger for larger dimensions of the cross-section of the inlets. Having a smaller dimension of the cross-section of the inlets means that a larger number of inlets may be used, which may imply that the pressure drop may decrease.

Using the smallest dimension of the cross-section of the inlets in the range of 20 - 300 $\mu$m, and in particular in the range of 100 - 200 $\mu$m facilitates that the collecting device will provide a collection efficiency of at least 50% for particles having a diameter larger than 300 nm and also facilitates that the pressure drop in the flow of air passing the collecting device will be lower than 3 kPa, even lower than 1.5 kPa, at a flow rate of 0.5 liters per second.

The dimensions of the inlets apply at an end of the inlet into the particle collection chamber. Thus, the inlets may be tapered towards a smaller cross-section at an interface to the particle collection chamber.

The cross-section of the inlets can be identical in two perpendicular directions. For instance, the cross-section of the inlet may have a shape of a circle or a square, which means that the smallest dimension of the inlet is defined by a size of a diameter and a side, respectively.

However, the cross-section of the inlets need not be identical in two perpendicular directions. Rather, the cross-section of the inlet may have a shape of an ellipse or a rectangle, which means that the smallest dimension of the inlet is defined by the minor axis and the short side, respectively.

It should be realized that the pressure drop experienced by the flow of air passing through the collecting device is mostly dependent on the smallest dimension of the cross-section of the inlet. Further, the collection efficiency of the particles need not be greatly impaired by a largest dimension of the cross-section of the inlet being substantially larger than the smallest dimension. For instance, the inlets may be designed as rectangular slits having a very large length and a small width, wherein the width is in the range of 20 - 300 $\mu$m, such as in the range of 100 - 200 $\mu$m. Use of rectangular slits may allow for an efficient arrangement of the inlets and outlets such that a small footprint (area of the first and/or second layer) of the collecting device may be achieved.

According to an embodiment, a number of inlets is larger than 100, such as larger than 500, such as 1000 - 2000 inlets.

The number of inlets used may control a total flow rate through the collecting device, and an associated pressure drop experienced by the flow of air. Since the inlets may need to have a small diameter size (in order to have a high collection efficiency of particles) and it is desired that the supply pressure to be provided by the person breathing into the collecting device is relatively low, the number of inlets may need to be high in order to provide a sufficient flow rate of air through the collecting device. A sample collected through breathing may desirably be collected within a minute, as it would ensure that the sampling may be quickly performed. Also, a short time for collecting the sample may ensure that the person providing the sample is not exhausted by providing the sample. Thus, the flow rate through the collecting device should be sufficiently high such that a relatively large volume of air may be sampled. Therefore, the number of inlets may need to be quite large, such as more than 100, or more than 500. It should also be realized that with a smaller inlet diameter being used, the number of inlets should be larger in order to maintain the total flow rate through the collecting device. The number of inlets to be used may also depend on a shape of the cross-section of the inlets.

Having a large number of inlets may also ensure that the inlets are distributed over an entire area of the first layer with a small distance between adjacent inlets. This may ensure that a large collection efficiency may be provided using a small footprint of the collecting device.

According to an embodiment, a length of the inlets is in a range of 20 - 500 μm, such as in a range of 50 - 300 μm.

The length of the inlet may be selected so as to ensure that a structural stability of the collecting device is provided, as the length of the inlet will also correspond to a thickness of the first layer, through which the inlets extend. Thus, a larger thickness of the first layer, and hence a larger length of the inlets, may ensure that a mechanical stability of the collecting device is provided.

If the particle collection chamber is filled with a liquid reagent after collection of particles, the liquid reagent may also be filled into the inlets and outlets. Hence, the length of the inlets also affects an overall liquid volume which will fill the particle collection chamber. In order to have a small volume, the length of the inlets should not be too long.

According to an embodiment, a cross-section of the inlets is circular or rectangular.

Such cross-sections are suitably used in the collecting device, but it should be realized that other shapes of the cross-section may be envisaged.

Inlets having a circular cross-section may be relatively easily manufactured. Inlets having a rectangular cross-section may be used for forming slit-shaped inlets. This may be useful for providing an efficient arrangement of the inlets and outlets such that a small footprint of the collecting device may be achieved.

According to an embodiment, a smallest dimension of a cross-section of the outlets is in a range of 20 - 400 μm, such as in a range of 100 - 300 μm.

The size and shape of the outlets may be chosen in relation to at least a pressure drop to be experienced by the flow of air when passing through collecting device. The size and shape of the outlets may also be chosen in relation to the size and shape of the inlets such that the outlets and the inlets may be staggered and such that the inlets may be arranged directly above an unbroken part of the first surface of the second layer wherein capturing of airborne particles occur.

In order to ensure that the pressure drop to be experienced by the flow of air when passing through the collecting device is small, the smallest dimension of the cross-section of the outlets may need to be large (and not differ substantially from dimensions of other cross-sections passed by the flow of air in a path through the collecting device). However, it should be realized that the pressure drop need not necessarily be larger for larger dimensions of the cross-section of the inlets. Having a smaller dimension of the cross-section of the outlets means that a larger number of outlets may be used, which may imply that the pressure drop may decrease.

Using the smallest dimension of the cross-section of the outlets in the range of 20 - 400 μm, and in particular in the range of 100 - 300 μm facilitates that the outlets may fit the sizes of the inlets in a collecting device having a collection efficiency of at least 50% for particles having a diameter larger than 300 nm and also facilitates that the pressure drop in the flow of air passing the collecting device will be lower than 3 kPa, even lower than 1.5 kPa, at a flow rate of 0.5 liters per second. Since the dimension of the outlets may not directly influence the collection efficiency of the collecting device, the outlets may in some embodiments be larger than the inlets.

The dimensions of the outlets apply at an end of the outlet at the particle collection chamber. Thus, the outlets may be tapered towards a smaller cross-section at an interface to the particle collection chamber.

The cross-section of the outlets can be identical in two perpendicular directions. For instance, the cross-section of the outlet may have a shape of a circle or a square, which means that the smallest dimension of the outlet is defined by a size of a diameter and a side, respectively.

However, the cross-section of the outlets need not be identical in two perpendicular directions. Rather, the cross-section of the outlet may have a shape of an ellipse or a rectangle, which means that the smallest dimension of the outlet is defined by the minor axis and the short side, respectively. Typically, the shapes of the outlets may be the same as the shapes of the inlets, albeit possibly with other dimensions, such that both the inlet and the outlets may e.g. be circular.

According to an embodiment, a number of outlets is larger than 100, such as larger than 500, such as 1000 - 2000 outlets.

The number of outlets used may fit a total flow rate provided into the collecting device from the inlets and an associated pressure drop experienced by the flow of air. The outlets may be arranged such that the flow of air entering the particle collection chamber through an inlet will be able to escape the particle collection chamber through an outlet without necessarily passing another adjacent inlet. This may ensure that behavior of the flow of air is not changed in the particle collection chamber so that no large pressure drop occurs in the particle collection chamber.

Hence, the number of outlets may be similar to the number of inlets, although the number of outlets need not be exactly the same as the number of inlets.

Having a large number of outlets may also ensure that the outlets are distributed over an entire area of the second layer with a small distance between adjacent outlets.

According to an embodiment, a length of the outlets is in a range of 20 - 500 $\mu$m, such as in a range of 100 - 300 $\mu$m.

The length of the outlet may be selected so as to ensure that a structural stability of the collecting device is provided, as the length of the outlet will also correspond to a thickness of the second layer, through which the inlets extend. Thus, a larger thickness of the second layer, and hence a larger length of the outlets, may ensure that a mechanical stability of the collecting device is provided.

If the particle collection chamber is filled with a liquid reagent after collection of particles, the liquid reagent may also be filled into the inlets and outlets. Hence, the length of the outlets also affects an overall liquid volume which will fill the particle collection chamber. In order to have a small volume, the length of the outlets should not be too long.

According to an embodiment, the first layer and the second layer are spaced apart by a gap in a range of 10 - 150 $\mu$m, such as in a range of 20 - 100 $\mu$m.

A small gap between the first and second layers is beneficial for providing a high collection efficiency (as the first surface of the second layer is close to the inlets at which the flow of air enters the particle collection chamber). A small gap also facilitates having a small volume of the particle collection chamber. Thus, it may be desired that as small gap as possible is provided.

However, a small gap may also imply that the pressure drop experienced by the flow of air passing through the collecting device is relatively high. Thus, in order to not cause too large pressure drop, a larger gap may be desired.

Thus, in order to balance the collection efficiency and volume of the particle collection chamber with the pressure drop, the gap may be in the range of 10 - 150 $\mu$m. Preferably, the gap may be in the range of 20 - 100 $\mu$m.

Further, the gap between the first and the second layers may be related to the size of the inlet in order to avoid large pressure drops when the flow of air passes through the collecting device. According to an embodiment, a ratio between the gap between the first and the second layers and a diameter of a circular inlet is in a range of 0.1 - 0.6.

According to an embodiment, a projection of the inlets onto the first surface of the second layer form a hexagonal arrangement of the inlets surrounding each outlet.

The hexagonal arrangement may be a suitable arrangement of the inlets and the outlets. However, it should be realized that other arrangements are also possible.

The arrangement of the outlets and inlets may be provided such that the flow of air entering the particle collection chamber through an inlet will be able to escape the particle collection chamber through an outlet without necessarily passing another adjacent inlet. This may ensure that behavior of the flow of air is not changed in the particle collection chamber so that no large pressure drop occurs in the particle collection chamber.

According to an embodiment, a projection of an inlet onto the first surface of the second layer is configured not to overlap with a closest neighbor outlet, such as a lateral separation of an edge of the projection of the inlet to an edge of the closest neighbor outlet being at least 20 $\mu$m.

When there is no overlap between edges of the inlet and outlets, the flow of air needs to change direction through the particle collection chamber, which may be advantageous in providing an efficient capturing of particles. The inlets and outlets may be arranged such that edges of the projection of the inlet and the closest neighbor outlet touch, i.e. that there is no lateral separation. However, according to an embodiment, there is at least a lateral separation of 20 $\mu$m. This may be beneficial for manufacturing of the collecting device.

According to a second aspect, there is provided a method for collection of airborne particles from a flow of air, said method comprising: receiving the flow of air onto a first layer of a collecting device, wherein the first layer comprises a plurality of inlets extending through the first layer; passing the flow of air through the inlets into a particle collection chamber between the first layer and a second layer of the collecting device spaced apart from the first layer; capturing airborne particles in the flow of air entering the particle collection chamber by impaction of airborne particles on a first surface of the second layer, wherein ends of the inlets are configured to face the first surface of the second layer; passing the flow of air out of the particle collection chamber through outlets extending through the second layer of the collecting device; wherein the inlets and outlets are staggered such that the center axes of the inlets are displaced from the center axes of the outlets; wherein the collecting device is configured such that the flow of air experiences a pressure drop when passing the collecting device, the pressure drop being lower than 3 kPa at a flow rate of 0.5 liters per second.

Effects and features of this second aspect are largely analogous to those described above in connection with the first aspect. Embodiments mentioned in relation to the first aspect are largely compatible with the second aspect.

Thanks to the method, an efficient capturing of airborne particles may be provided in the particle collection chamber without requiring a high supply pressure to be provided by the person breathing through the collecting device.

As used herein, the term "flow of air experiences a pressure drop when passing the collecting device" should be construed as the pressure drop experienced along an entire path from an inlet of the collecting device (defined by open ends of the inlets allowing the flow of air to enter the collecting device) to an outlet of the collecting device (defined by open ends of the outlets allowing the flow of air to leave the collecting device). Thus, the experienced pressure drop may occur in the inlets, in the particle collection chamber and in the outlets or in a combination thereof.

It should be realized that the pressure drop depends on the flow rate through the collecting device. The flow rate of the flow of air through the collecting device based on air exhaled by the human being will depend on the exhalation flow rate provided by the human being. Thus, the actual pressure drop that will occur in the collecting device will depend on the exhalation flow rate that is provided. However, the collecting device used in the method is designed such that, when a flow rate of 0.5 liters per second is received through the collecting device, the pressure drop is lower than 3 kPa.

The method may facilitate an easy and intuitive way of collecting samples through self-test by persons, thereby promoting more frequent testing of people. In this regard, the use of the method for screening of people may allow people to be screened several times per week, such as every day, such that viral load peaks may be identified. If testing is done only once a week, the viral load peak may be missed such that the testing fails to detect persons having a stage of a disease in which the person is highly infectious. Hence, very frequent testing may be required in order to identify highly infectious people and prevent spreading of a disease.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a schematic cross-sectional view of a sample collector including a collecting device for collection of airborne particles according to an embodiment.
Fig. 2 is a schematic cross-sectional view of a collecting device according to an embodiment.

Fig. 3 is a graph defining efficiency of capturing of particles through impaction and an explanatory cross-section of an inlet nozzle providing air flow for capturing particles through impaction.
Figs 4-5 are schematic views illustrating arrangement of inlets and outlets of the collecting device according to different embodiments.
Figs 6-8 are schematic cross-sectional views of the collecting device according to different embodiments.
Fig. 9 is a flowchart of a method according to an embodiment.

Detailed description

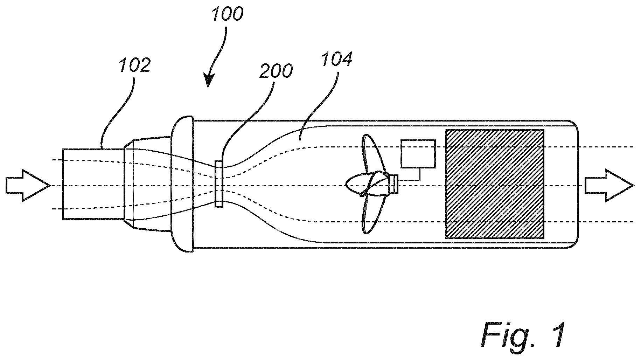

Referring now to Fig. 1, a collecting device 200 for collection of airborne particles from a flow of air is shown in relation to a sample collector 100. It should thus be realized that according to an embodiment, the collecting device 200 may be arranged in a sample collector 100 which provides an interface for allowing a flow of air from a human being to be provided through the collecting device 200. However, it should be realized that the collecting device 200 may be arranged to receive the flow of air in different manners and need not necessarily be mounted in a sample collector 100 or any other apparatus.

The sample collector 100 may be used for capturing airborne particles, such as aerosols and/or droplets in the flow of air exhaled by the human being. Thanks to capturing airborne particles, analysis of the airborne particles in the exhaled breath may be performed. This may be used for determining whether the human being carries a disease, which is spread through droplets and aerosols produced during normal breathing, talking, coughing, and sneezing. For instance, the capturing of airborne particles using the sample collector 100 may be used for screening whether a person is infected by influenza or severe acute respiratory syndrome coronavirus 2 (SARS-CoV-2). Thanks to the sample collector 100 capturing a sample based on an exhaled breath, the capturing of a sample from a person may be performed with minimal discomfort to the person.

The sample collector 100 may comprise a mouthpiece 102 to be inserted into the mouth of the person and through which the person exhales to provide a flow of air 104 through the sample collector 102.

The flow of air 104 may be guided through the sample collector 102 so as to pass the collecting device 200. The collecting device 200 is configured to capture airborne particles from the flow of air 104 through impaction in a particle collection chamber of the collecting device 200. The collecting device 200 may be configured to capture airborne particles with high efficiency and may further allow analysis of the collected airborne particles.

Analysis of the collected airborne particles may involve sample preparation by providing a reagent to the particle collection chamber for allowing reactions to take place in the particle collection chamber. The reactions may further be controlled by providing further influence on the particle collection chamber, such as by heating and/or cooling the sample in the particle collection chamber.

Furthermore, analysis of the collected airborne particles may be performed while the collecting device 200 is maintained in the sample collector 100. This implies that a risk of spreading of disease by opening of the sample collector 100 may be avoided.

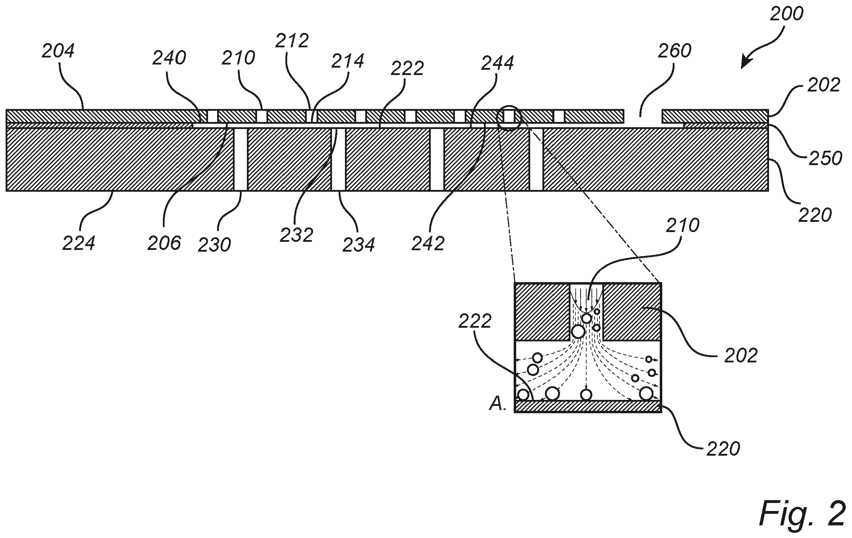

Referring now to Fig. 2, the collecting device 200 according to an embodiment will be further described.

The collecting device 200 comprises a first layer 202 and a second layer 220. The first layer 202 and the second layer 220 are arranged to be spaced apart for defining a particle collection chamber 240 between the first layer 202 and the second layer 220.

The first layer 202 and the second layer 220 may each be formed from a semiconductor or semiconductor-based material, such as silicon or silicon dioxide. This may facilitate manufacturing of the collecting device 200, since small dimensions of the collecting device may be advantageously provided by semiconductor manufacturing processes.

The first layer 202 comprises a first surface 204 configured to receive a flow of air, which may be the flow of air 104 in the sample collector 100 as described above. The first layer 202 also comprises a second surface 206 facing the second layer 220. The first layer 202 further comprises a plurality of inlets 210 having a first end 212 at the first surface 204 of the first layer 202 and a second end 214 at the second surface 206 of the first layer 204.

The second layer 220 comprises a first surface 222 facing the first layer 202 and a second surface 224 at which the flow of air may be output from the collecting device 200 after having passed the collecting device 200. The second layer 220 further comprises a plurality of outlets 230 having a first end 232 at the first surface 222 of the second layer 220 and a second end 234 at the second surface 224 of the second layer 220.

The inlets 210 are configured to extend through the first layer 202 for transporting the flow of air 104 through the first layer 202 from the first end 212 to the second end 214. The second ends 214 of the inlets 210 are configured to face the first surface 222 of the second layer 220. Thus, when the flow of air 104 passes through the inlets 210, the flow of air 104 will impinge on the first surface 222 of the second layer 220 such that airborne particles may be collected on the first surface 222 of the second layer 220 and, hence, in the particle collection chamber 240, by impaction.

The particle collection chamber 240 has a first side 242 and a second side 244, wherein the first side 242 is defined by the second surface 206 of the first layer 202 and the second side 244 is defined by the first surface 222 of the second layer 220. The particle collection chamber 240 may further be defined by side surfaces formed in a spacer material 250 between the first layer 202 and the second layer 220.

The spacer 250 can either be a glue, double sided-adhesive tape, or in case of silicon/glass layers 202, 220, the spacer 250 can be integrated into one of the layer materials to enable anodic, fusion, or laser bonding of the first layer 202 and the second layer 220.

In another embodiment, not shown in Fig. 2, the inlets 210 may be configured to protrude from second surface 206 of the first layer 202 to extend further into the particle collection chamber 240. This may imply that the second ends 214 of the inlets 210 are closer to the first surface 222 of the second layer 220 so as to improve capturing of particles by impaction while ensuring that a volume of the particle collection chamber 240 is not too small.

The inlets 210 and the outlets 230 are arranged in a staggered arrangement. This implies that center axes of inlets 210 and outlets 230 are not aligned. Hence, the flow of air 104 passing through the inlets 210 into the particle collection chamber 240 will at least slightly change direction through the particle collection chamber 240 before the flow of air 104 may exit the particle collection chamber 240 through the outlets 230.

Thanks to the inlets 210 and the outlets 230 being staggered, the inlets 210 are arranged directly above the first surface 222 of the second layer 220 wherein capturing of airborne particles occur. The inlets 210 are arranged such that there is at least no opening in the first surface 222 of the second layer 220 corresponding to an outlet 230 at a projection of the center axes of the inlets 210 onto the first surface 222 of the second layer 220. As shown in Fig. 2, the inlets 210 and the outlets 230 are arranged such that the entire inlet 210 is projected on an area of the first surface 222 wherein no openings corresponding to outlets 240 are provided. Thus, airborne particles may be captured at the first surface 222 of the second layer 220, while the flow of air 104 through the inlets 210 changes direction to follow the first surface 222 and then escape the particle collection chamber 240 through the outlets 230.

As shown in Fig. 2, the inlets 210 may extend perpendicularly in relation to the first layer 202. Further, the outlets 240 may extend perpendicularly in relation to the second layer 220. With the first and second layer 202, 220 being parallel, this implies that the inlets 210 and the outlets 240 are parallel, arranged with the central axes displaced in relation to each other. This is a suitable arrangement for ensuring that the inlets 210 and outlets 240 are staggered and that the flow of air 104 is forced to change direction through the collecting device 200.

Thanks to the flow of air 104 being forced to change direction, momentum of airborne particles having a certain size will cause the airborne particles not to follow the flow of air 104 in its change of direction and instead the particles will be captured on the collection surface formed by the first surface 222 of the second layer 220. The capturing of airborne particles may involve capturing of aerosols but may also or alternatively involve capturing of larger droplets in the flow of air.

Collection of particles in the collecting device 200 is further illustrated in the enlarged insert A of Fig. 2, illustrating that airborne particles will be captured by impaction on the first surface 222 of the second layer 220, before the flow of air 104 proceeds to outlets 230 extending through the second layer 220.

The collecting device 200 may further be configured to provide optical access for performing a measurement, based on light, of airborne particles collected in the particle collection chamber 240.

The measurement may be performed based on light that is passed through at least one of the first and second layers 202, 220. Thus, the first layer 202 and/or the second layer 220 may be transparent or translucent to provide optical access to the particle collection chamber 240. However, according to another embodiment, optical access is provided through the inlets 210 and/or the outlets 230.

In addition to the inlets 210 and outlets 230, a liquid access port 260 providing a reagent inlet may be provided in the collecting device 200. The liquid access port 260 may extend through the first layer 202 as shown in Fig. 2, but may alternatively extend through the second layer 220 instead. The liquid access port 260 enables filling of the particle collection chamber 240 with a liquid reagent, such as a polymerase chain reaction (PCR) reagent.

The collecting device 200 may further be configured to allow a sample in the particle collection chamber 240 to be heated and/or cooled, possibly in numerous iterations, in order to prepare the sample for analysis. For instance, thermal energy may be provided to the particle collection chamber 240 for thermal lysis to expose RNA of SARS-CoV-2 in the captured particles, converting the RNA to DNA using reverse transcriptase based on the reagent and providing thermal cycling for amplification of the DNA using quantitative PCR.

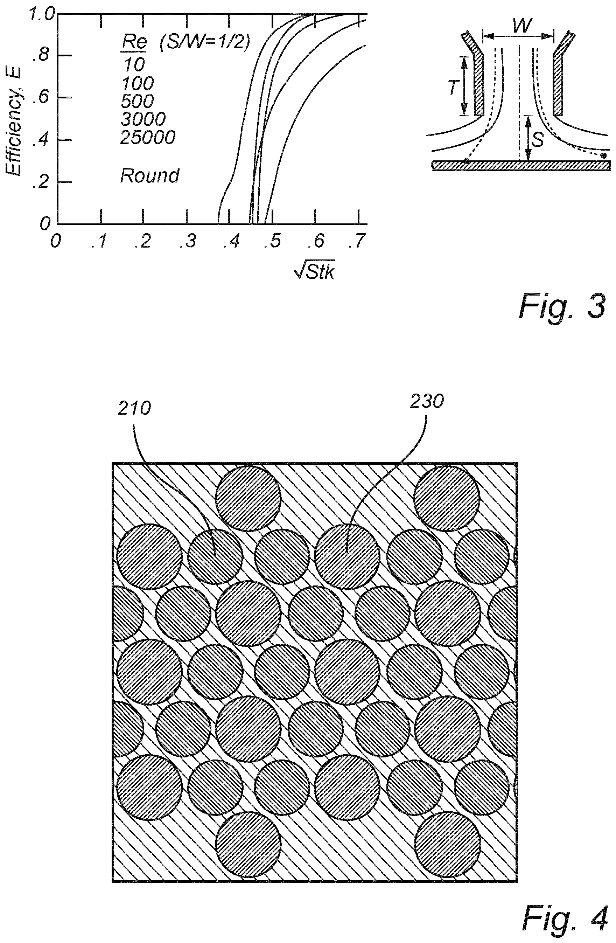

Referring now to Fig. 3, design of the collecting device 200 for providing an efficient capturing of particles will be discussed.

Fig. 3 is based on a figure from Marple and Willeke, "Impactor Design", Atmospheric Environment, vol. 10 (10), 1976, pp. 891-896.

The collection efficiency, the percentage of particles that will impact the plate, is a strong function of the Stokes number and Reynolds number. The Stokes number is defined as:

$$Stk = \frac{4\rho_p Q C_c d_p^2}{9\pi N \mu_g W^3}$$

where $\rho_p$ is the density of the particle, $Q$ is the total volumetric flow rate for an array of inlets, $C_c$ is the Cunningham correction factor (taken as 1 for all subsequent calculations), $d_p$ is the particle diameter, $N$ is the number of inlets in the array, $\mu_g$ is the dynamic viscosity of air, and $W$ is the inlet diameter. For the above study, distances $T$ and $S$ as indicated in Fig. 3 are $T = 2 W$ and $S = W / 2$. The Reynolds number is defined as:

$$Re = \frac{4\rho_p Q}{\pi N \mu_g W}$$

According to the Fig. 3, in order to capture 50% of the particles at a Reynolds number of $Re = 100$, then $\sqrt{\frac{4\rho_p Q C_c d_p^2}{9\pi N \mu_g W^3}} \approx 0.48$. Note that for increasing particle diameters, $\sqrt{Stk}$ increases and higher collection efficiencies are obtained. Conversely, small particles are more difficult to capture than large particles.

According to an embodiment, the collecting device 200 is intended to be used for collecting a sample from exhaled breath from a person and then perform subsequent analysis on the collecting device 200 to screen for possible infectious disease(s). The analysis, for instance, could be end-point or quantitative reverse transcriptase polymerase chain reaction (RT-PCR) for an RNA virus like SARS-CoV-2.

It is desired to have a high collection efficiency of approximately 50% for particle diameters down to 300 nm. This is to ensure that enough sample can be collected to have reasonably sensitive results for detection of the biological agent of interest from a reasonably small sampling of exhaled breath from the patient.

Furthermore, it is intended that the person exhales through the collecting device 200. In order to ensure that the person can properly blow through the collecting device 200 without discomfort, the collecting device 200 is designed such that the pressure drop experienced by the flow of air 104 when passing through the collecting device 200 is lower than 3 kPa at a flow rate of 0.5 liters per second. Further, the person that is to exhale through the collecting device 200 may suffer from disease such that the person has a shortage of breath. In this respect, the collecting device 200 is preferably designed such that the pressure drop experienced by the flow of air 104 when passing through the collecting device 200 is lower than 1.5 kPa at a flow rate of 0.5 liters per second.

The desired pressure drop to be provided through the collecting device 200 may be used for designing the collecting device 200. Thus, dimensions of the collecting device 200 may be set in order to ensure that the pressured drop is within the desired range.

The pressure drop is dependent on dimensions of airway channels (inlets 210, particle collection chamber 240 and outlets 230) through which the flow of air 104 passes when passing the collecting device 200. However, the inlets 210 are also designed in order to provide a desired collection efficiency of particles in the particle collection chamber 240. Further, the particle collection chamber 240 may also be designed such that the volume of the particle collection chamber 240 is relatively small. The small volume of the particle collection chamber 240 facilitates performing fast analysis of a sample in the particle collection chamber 240.

The particle collection chamber 240 may typically be filled with a reagent after collection of particles in the particle collection chamber 240. Then, the sample in the particle collection chamber 240 may need to be heated and/or cooled, possibly in numerous iterations, in order to prepare the sample for analysis. Having a small volume of the particle collection chamber 240 implies that steps of heating and/or cooling the sample in the particle collection chamber 240 may be quickly performed.

Thus, the collecting device 200 may further be designed such that the volume of the particle collection chamber 240 is smaller than 30 μl. Preferably, the volume of the particle collection chamber 240 should be smaller than 20 μl.

Further, the collecting device 200 may further be designed to provide a collection efficiency of at least 50% for particles having a diameter larger than 300 nm when a flow rate the flow of air 104 passing the collecting device 200 is 0.5 liters per second.

The collecting device 200 is configured to meet at least the requirement relating to pressure drop being less than 3 kPa for a flow rate of the flow of air 104 of 0.5 liters per second, but is preferably also configured to meet the requirement relating to volume of the particle collection chamber 240 being smaller than 30 μl and the requirement relating to the collection efficiency.

The smallest dimension of a cross-section of the inlets 210 may be in a range of 20 - 300 μm. Preferably, the smallest dimension of the cross-section of the inlets 210 is in a range of 100 - 200 μm.

The size and shape of the inlets 210 may be set in relation to at least a desired efficiency of collection of particles, since the inlets 210 highly affect the efficiency of collection of particles. Then, other features of the collecting device 200 may be related to the size and shape of the inlets 210 such that at least the desired pressure drop is provided.

As indicated in the above discussion, the smallest dimension of the cross-section of the inlets 210 needs to be small in order to provide efficient collection of particles having a small diameter. However, a small dimension of the cross-section of the inlets 210 may imply that a large pressure drop is provided.

Using the smallest dimension of the cross-section of the inlets in the range of 20 - 300 μm, and in particular in the range of 100 - 200 μm facilitates that the collecting device will provide a collection efficiency of at least 50% for particles having a diameter larger than 300 nm and also facilitates that the pressure drop in the flow of air passing the collecting device will be lower than 3 kPa, even lower than 1.5 kPa, at a flow rate of 0.5 liters per second.

The cross-section of the inlets 210 may be circular. This may be suitable for manufacturing of the collecting device 200. Also, it may ensure that a symmetric behavior of the flow of air 104 flowing through the inlets 210 is provided around the central axis of the inlet 210. For a circular cross-section, the smallest dimension of the inlet 210 is the diameter of the inlet 210. However, it should be realized that other shapes of the cross-section of the inlets 210 are conceivable. For instance, the inlets 210 may be rectangular and may even be in the form of elongate rectangular slits.

The number of inlets 210 used may control a total flow rate through the collecting device 200, and an associated pressure drop experienced by the flow of air 104. In view of the cross-sectional dimension of the inlets 210, the number of inlets 210 may be selected to provide a sufficient flow rate of air through the collecting device 200. With large inlets 210, each inlet 210 supports a relatively large flow rate, whereas small inlets 210 support a relatively small flow rate. Thus, the number of inlets 210 may need to be larger for smaller inlets 210.

Since the size of the inlets 210 is preferably small for a large collection efficiency as discussed above, the number of inlets 210 may need to be quite large, such as more than 100, or more than 500, such as in a range of 1000-2000.

The length of the inlets 210 corresponds to a thickness of the first layer 202. The thickness of the first layer 202 may affect a stability of the collecting device 200, together with a thickness of the second layer 220 and a gap between the first 202 and second layers 220, such that the thickness of the first layer 202 should not be too small and, hence the length of the inlets 210 should not be too small. However, a liquid reagent filled into the particle collection chamber 240 may also be filled into the inlets 210 and the outlets 230, such that the length of the inlets 210 affect an overall liquid volume filling the particle collection chamber 240. Thus, the length of the inlets 210 should not be too large.

Therefore, the length of the inlets 210 of the collecting device 200 may be in a range of 20 - 500 μm. Preferably, the length of the inlets 210 is in a range of 50 - 300 μm.

A small gap between the first layer 202 and the second layer 220 is beneficial for providing a high collection efficiency. A small gap also facilitates having a small volume of the particle collection chamber 240. However, a small gap may also imply that the pressure drop experienced by the flow of air 104 passing through the collecting device 200 is relatively high.

Thus, in order to balance the collection efficiency and the volume of the particle collection chamber 240 with the pressure drop experienced by the flow of air 104, the gap may be in the range of 10 - 150 μm. Preferably, the gap may be in the range of 20 - 100 μm.

Further, the gap may be related to the size of the inlets 210 in order to avoid large pressure drops when the flow of air 104 passes through the collecting device 200.

A size ratio between the gap and a diameter of a circular inlet 210 may be in a range of 0.1 - 0.6.

A projection of the inlets 210 along the respective center axes of the inlets 210 onto the first surface 222 of the second layer 220 may be arranged not to intersect with the first ends 232 of the outlets 230 in the second layer 220. This implies that the flow of air 104 following an extension of the inlets 210 will impinge on a part of the first surface 222 of the second layer 220 allowing particles to be captured at the first surface 222. However, it should be realized that even if there is an overlap between the projection of the inlets 210 and the outlets 230, the flow of air 104 will at least slightly change direction by the center axes of the inlets 210 and outlets 230 not being aligned. Therefore, collection of particles may be provided in the particle collection chamber 240 even if there is an overlap between the projection of the inlets 210 and the outlets 230.

However, for ensuring that a high collection efficiency is provided, the projection of the inlets 210 and the outlets 230 may be configured not to overlap. In order to cause a larger change of direction of the flow of air 104, there may be a lateral separation of at least 20 µm between an edge of the projection of an inlet 210 and an edge of the closest neighbor outlet.

The size and shape of the outlets 230 may be set in relation to the size and shape of the inlets 210 such that the outlets 230 may be symmetrically arranged in relation to the inlets 210 and that the flow of air 104 passes through inlets 210 and outlets 230 of similar dimensions. This may facilitate that a low pressure drop is experienced by the flow of air 104 when passing the collecting device 200. Further, the size and shape of the outlets 230 may be set in relation to the size and shape of the inlets 210 such that the inlets 210 and the outlets 230 may be staggered.

Using the smallest dimension of the cross-section of the outlets 230 in the range of 20 - 400 µm and in particular in the range of 100 - 300 µm facilitates that the pressure drop in the flow of air passing the collecting device will be lower than 3 kPa, even lower than 1.5 kPa, at a flow rate of 0.5 liters per second.

The outlets 230 may be slightly larger than the inlets 210 since the outlets 230 are not directly involved in the particle collection.

The cross-section of the outlets 230 may be circular. This may be suitable for manufacturing of the collecting device 200. Also, it may ensure that a symmetric behavior of the flow of air 104 flowing through the outlets 230 is provided around the central axis of the outlet 230. For a circular cross-section, the smallest dimension of the outlet 230 is the diameter of the outlet 230. However, it should be realized that other shapes of the cross-section of the outlets 230 are conceivable. For instance, the outlets 230 may be rectangular and may even be in the form of elongate rectangular slits.

The number of outlets 230 used may fit a total flow rate provided into the collecting device 200 from the inlets 210 and an associated pressure drop experienced by the flow of air 104. The outlets 230 may be arranged such that the flow of air 104 entering the particle collection chamber 240 through an inlet 210 will be able to escape the particle collection chamber 240 through an outlet 230 without necessarily passing another adjacent inlet 210. This may ensure that behavior of the flow of air 104 is not changed in the particle collection chamber 240 so that no large pressure drop occurs in the particle collection chamber 240.

With large outlets 230, each outlet 230 supports a relatively large flow rate, whereas small outlets 230 support a relatively small flow rate. Thus, the number of outlets 230 may need to be larger for smaller outlets 230.

The number of outlets 230 may be similar to the number of inlets 210, although the number of outlets 230 need not be exactly the same as the number of inlets 210. Since the outlets 230 may be slightly larger than the inlets 210, as discussed above, the number of outlets 230 may be slightly smaller than the number of inlets 210.

However, the size of the outlets 230 is preferably small, such that the number of outlets 230 may need to be quite large, such as more than 100, or more than 500, such as in a range of 1000-2000.

The length of the outlets 230 corresponds to a thickness of the second layer 220. The thickness of the second layer 220 may affect a stability of the collecting device 200, together with a thickness of the first layer 202 and a gap between the first 202 and second layers 220. In particular, as the second layer 220 may function as a substrate of the collecting device 200, the thickness of the second layer 220 should not be too small. Also, in order to ensure an overall stability of the collecting device 200, a combined thickness of the second layer 220, the gap between the first 202 and second layers 220, and the first layer 202, may be at least 450 µm. However, a liquid reagent filled into the particle collection chamber 240 may also be filled into the inlets 210 and the outlets 230, such that the length of the outlets 230 affect an overall liquid volume filling the particle collection chamber 240. Thus, the length of the outlets 230 should not be too large.

Therefore, the length of the outlets 230 of the collecting device 200 may be in a range of 20 - 500 µm. Preferably, the length of the outlets 230 is in a range of 100 - 300 µm.

Referring now to Fig. 4, an arrangement of the inlets 210 and the outlets 230 according to an embodiment will be discussed. Fig. 4 illustrates the arrangement of a projection of the shape and size of the inlets 210 at the second end 214 onto the first surface 222 of the second layer 220. Fig. 4 further illustrates the first ends 232 of the outlets 230 such that the relative arrangement of the inlets 210 and the outlets 230 is shown.

Six inlets 210 are arranged around each outlet 230 with an even distribution of the six inlets 210 around a circumference of the outlet 230. Thus, the inlets 210 form a hexagonal arrangement surrounding the outlet 230.

Further, each inlet 210 is arranged between three outlets 230 with the three outlets 230 separated by an equal lateral separation in relation to the inlet 210.

The lateral separation between an edge of the projection of the inlet 210 and edges of the three outlets 230 closest to the inlet 210 may be smaller than a lateral separation between adjacent inlets 210.

The arrangement of the inlets 210 and outlets 230 as shown in Fig. 4 provides that the flow of air 104 entering the particle collection chamber 240 through an inlet 210 will be able to escape the particle collection chamber 240 through an outlet 230 without necessarily passing another adjacent inlet 210. This may ensure that behavior of the flow of air 104 is not changed in the particle collection chamber 240 so that no large pressure drop occurs in the particle collection chamber 240.

The arrangement of the inlets 210 and the outlets 230 further provides an efficient use of a footprint (area of the first layer 202 and/or the second layer 220) of the collecting device 200 while arranging the inlets 210 and the outlets 230 with no overlap.

However, it should be realized that the inlets 210 and the outlets 230 may be arranged in many other relations to each other. For instance, the projection of the inlets 210 and the outlets 230 may form a plurality of rows, wherein every other row is formed by inlets 210 and every other row is formed by outlets 230.

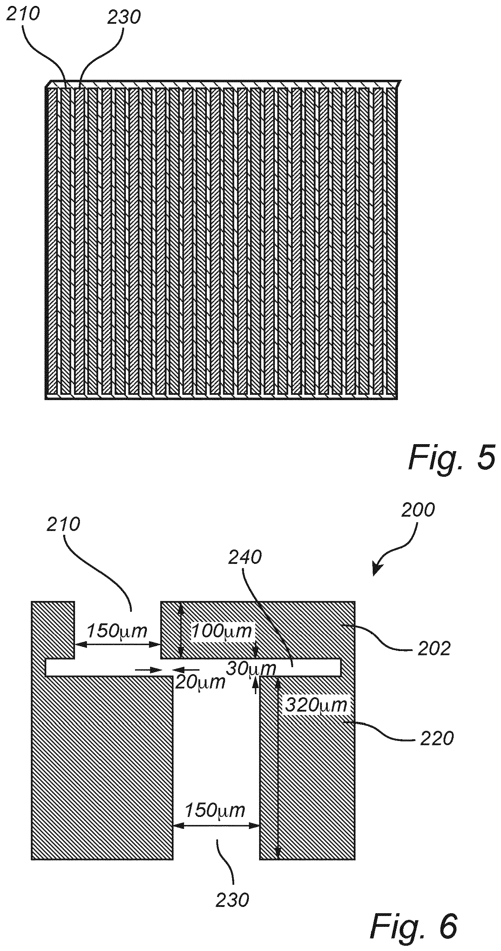

Referring now to Fig. 5, an arrangement of the inlets 210 and the outlets 230 according to another embodiment will be discussed. Fig. 5 also illustrates the arrangement of a projection of the shape and size of the inlets 210 at the second end 214 onto the first surface 222 of the second layer 220. Fig. 5 further illustrates the first ends 232 of the outlets 230 such that the relative arrangement of the inlets 210 and the outlets 230 is shown.

The arrangement of the inlets 210 and outlets 230 as shown in Fig. 5 also provides that the flow of air 104 entering the particle collection chamber 240 through an inlet 210 will be able to escape the particle collection chamber 240 through an outlet 230 without necessarily passing another adjacent inlet 210. This may ensure that behavior of the flow of air 104 is not changed in the particle collection chamber 240 so that no large pressure drop occurs in the particle collection chamber 240.

The collection efficiency of the particles is mainly determined by a smallest dimension of the cross-section of the inlet 210, such that a substantially larger dimension of the cross-section in a direction perpendicular to a direction of the smallest dimension could be used. Thus, as shown in Fig. 5, the inlets 210 may be designed as rectangular slits having a very large length and a small width. The width may be in the range of 20 - 300 μm, such as in the range of 100 - 200 μm in order to provide at least a desired efficiency of collection of particles and an acceptable pressure drop to be experienced by the flow of air 104 when passing through collecting device 200. The length of the rectangular slits may be in an order of millimeters, such as several or even tens of millimeters.

The use of rectangular slits may allow for an efficient arrangement of the inlets 210 and outlets 230 such that a small footprint of the collecting device 200 may be achieved.

Referring now to Figs 6-8, cross-sections of embodiments of the collecting device 200 are disclosed illustrating different sizes of parts of the collecting device 200. In each of the embodiments of Figs 6-8, circular cross-sections of the inlets 210 and outlets 230 are used. Also, the inlets 210 and outlets 230 are arranged in a hexagonal arrangement in relation to each other.

In the embodiment illustrated in Fig. 6, each inlet 210 has a diameter of 150 μm and a length of 100 μm. The collecting device 200 has 1600 inlets 210. Each outlet 230 has a diameter of 150 μm and a length of 320 μm. Further, there is a lateral separation of 20 μm between an edge of the projection of the inlet 210 and an edge of the adjacent outlet 230. There is a vertical gap between the first layer 202 and the second layer 220 of 30 μm.

Further, a width of the particle capturing chamber 240 is 10.6 mm and a volume of the particle capturing chamber 240 is 15.8 μl.

Using the collecting device 200 as described in relation to Fig. 6, the flow of air 104 experiences a pressure drop of 1.3 kPa at a flow rate of 0.5 liters per second. Further, the collecting device 200 is configured to provide a collection efficiency of 50% for particles having a diameter of 352 nm.

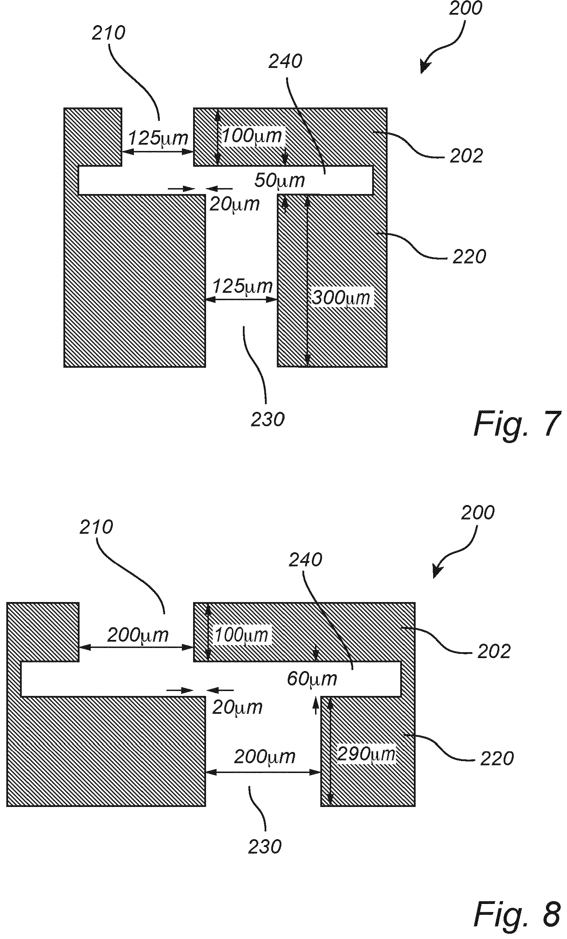

In the embodiment illustrated in Fig. 7, each inlet 210 has a diameter of 125 μm and a length of 100 μm. The collecting device 200 has 1561 inlets 210. Each outlet 230 has a diameter of 125 μm and a length of 300 μm. Further, there is a lateral separation of 20 μm between an edge of the projection of the inlet 210 and an edge of the adjacent outlet 230. There is a vertical gap between the first layer 202 and the second layer 220 of 50 μm.

Further, a width of the particle capturing chamber 240 is 8.1 mm and a volume of the particle capturing chamber 240 is 11.9 μl.

Using the collecting device 200 as described in relation to Fig. 7, the flow of air 104 experiences a pressure drop of 1.9 kPa at a flow rate of 0.5 liters per second. Further, the collecting device 200 is configured to provide a collection efficiency of 50% for particles having a diameter of 300 nm.

In the embodiment illustrated in Fig. 8, each inlet 210 has a diameter of 200 μm and a length of 100 μm. The collecting device 200 has 1015 inlets 210. Each outlet 230 has a diameter of 200 μm and a length of 290 μm. Further, is a lateral separation of 20 μm between an edge of the projection of the inlet 210 and an edge of the adjacent outlet 230. There is a vertical gap between the first layer 202 and the second layer 220 of 60 μm.

Further, a width of the particle capturing chamber 240 is 9.9 mm and a volume of the particle capturing chamber 240 is 20.2 μl.

Using the collecting device 200 as described in relation to Fig. 8, the flow of air 104 experiences a pressure drop of 0.7 kPa at a flow rate of 0.5 liters per second. Further, the collecting device 200 is configured to provide a collection efficiency of 50% for particles having a diameter of 500 nm.

Thus, it may be seen from the embodiments discussed in Figs 6-8 that a lower pressure drop experienced by the flow of air 104 passing the collecting device may be associated with a larger volume of the particle capturing chamber 240 and a larger size of the particles being captured with a collection efficiency of 50%.

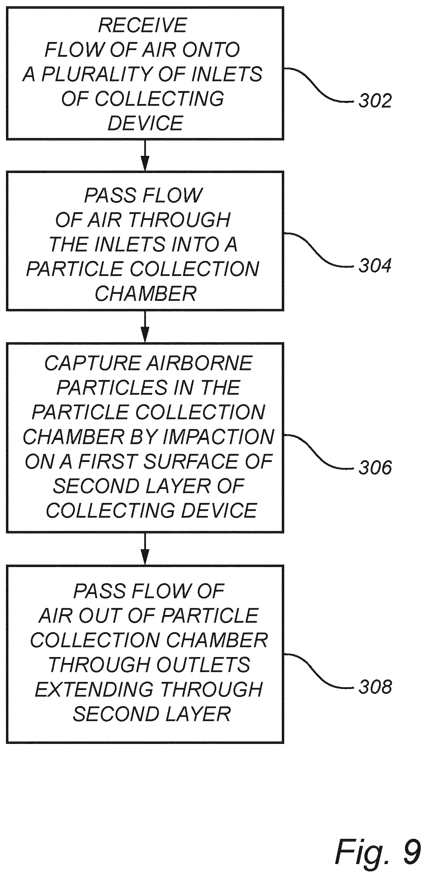

Referring now to Fig. 9, a method for collection of airborne particles exhaled by a human being will be described.

The method comprises receiving 302 a flow of air 104 from a human being onto a plurality of inlets 210 of a collecting device 200. The method further comprises passing 304 the flow of air 104 through the inlets 210 into a particle collection chamber 240.

The plurality of inlets 210 may extend through at least a first layer 202. The flow of air 104 may thus be received on a surface forming an interface between the collecting device 200 and an external environment outside the collecting device 200, e.g. an internal space in a sampling compartment of a sample collector 100. The flow of air 104 is passed by the plurality of inlets 210 from the external environment outside the collecting device 200 into the particle capturing chamber 240. The particle capturing chamber 240 may be defined between the first layer 202 and a second layer 220 of the collecting device 200 spaced apart from the first layer 202.

The method further comprises capturing 306 airborne particles in the flow of air 104 entering the particle collection chamber 240 by impaction of airborne particles on a first surface 222 of the second layer 220. The method further comprises passing 308 the flow of air 104 out of the particle collection chamber 240 through outlets 230 extending through at least the second layer 220 of the collecting device 200 to an external environment outside the collecting device 200.

The inlets 210 and the outlets 230 are staggered such that the center axes of the inlets 210 are displaced from the center axes of the outlets 230 and the center axes of the inlets 210 and the outlets 230 are not aligned. Thus, ends 214 of the inlets 210 are configured to face the first surface 222 of the second layer 220, such that particles are captured at the first surface 222 between the outlets 230. The method thus causes the flow of air 104 to be forced to change direction, such that momentum of particles having a certain size will cause the particles not to follow the flow of air 104 in its change of direction and instead the particles will be captured on the first surface 222.

The method passes the flow of air 104 through a collecting device 200 such that the flow of air 104 experiences a pressure drop when passing the collecting device 200, wherein the pressure drop is such that if the flow rate of the flow of air is 0.5 liters per second, the pressure drop will be lower than 3 kPa.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A collecting device (200) for collection of airborne particles from a flow of air, said collecting device (200) comprising:

   a first layer (202) and a second layer (220), wherein the first layer (202) and the second layer (220) are arranged to be spaced apart for forming a particle collection chamber (240) between the first (202) and the second layer (220), wherein the first layer (202) comprises a plurality of inlets (210) configured to extend through the first layer (202) for transporting the flow of air (104) therethrough into the particle collection chamber (240); wherein ends (214) of the inlets (210) are configured to face a first surface (222) of the second layer (220) for capturing airborne particles in the flow of air (104) entering the particle collection chamber (240) through the ends (214) of the inlets (210) by impaction of airborne particles on the first surface of the second layer (220); wherein the second layer (220) comprises a plurality of outlets (230) configured to extend through the second layer (220) for transporting the flow of air (104) therethrough out of the particle collection chamber (240); wherein the inlets (210) and outlets (230) are staggered such that the center axes of the inlets (210) are displaced from the center axes of the outlets (230); wherein the collecting device (200) is configured such that the flow of air experiences a pressure drop when passing the collecting device (200), the pressure drop being lower than 3 kPa at a flow rate of 0.5 liters per second.

2. The collecting device according to claim 1, wherein a volume of the particle collection chamber (240) is smaller than 30 μl, such as smaller than 20 μl.

3. The collecting device according to claim 1 or 2, wherein the collecting device (200) is configured such the pressure drop experienced by the flow of air when passing the collecting device (200) is lower than 1.5 kPa at a flow rate of 0.5 liters per second.

4. The collecting device according to any one of the preceding claims, wherein the collecting device (200) is configured to provide a collection efficiency of at least 50% for particles having a diameter larger than 300 nm when the collecting device (200) receives a flow of air (104) with a flow rate of 0.5 liters per second.

5. The collecting device according to any one of the preceding claims, wherein a smallest dimension of a cross-section of the inlets (210) is in a range of 20 - 300 μm, such as in a range of 100 - 200 μm.

6. The collecting device according to any one of the preceding claims, wherein a number of inlets (210) is larger than 100, such as larger than 500, such as 1000 - 2000 inlets.

7. The collecting device according to any one of the preceding claims, wherein a length of the inlets (210) is in a range of 20 - 500 μm, such as in a range of 50 - 300 μm.

8. The collecting device according to any one of the preceding claims, wherein a cross-section of the inlets (210) is circular or rectangular.

9. The collecting device according to any one of the preceding claims, wherein a smallest dimension of a cross-section of the outlets (230) is in a range of 20 - 400 μm, such as in a range of 100 - 300 μm.

10. The collecting device according to any one of the preceding claims, wherein a number of outlets (230) is larger than 100, such as larger than 500, such as 1000 - 2000 outlets.

11. The collecting device according to any one of the preceding claims, wherein a length of the outlets (230) is in a range of 20 - 500 μm, such as in a range of 100 - 300 μm.

12. The collecting device according to any one of the preceding claims, wherein the first layer (202) and the second layer (220) are spaced apart by a gap in a range of 10 - 150 μm, such as in a range of 20 - 100 μm.

13. The collecting device according to any one of the preceding claims, wherein a projection of the inlets (202) onto the first surface (222) of the second layer (220) form a hexagonal arrangement of the inlets (210) surrounding each outlet (230).

14. The collecting device according to any one of the preceding claims, wherein a projection of an inlet (210) onto the first surface (222) of the second layer (220) is configured not to overlap with a closest neighbor outlet (230), such as a lateral separation of an edge of the projection of the inlet (210) to an edge of the closest neighbor outlet (230) being at least 20 μm.

15. A method for collection of airborne particles from a flow of air, said method comprising:

receiving (302) a flow of air (104) onto a first layer (202) of a collecting device (200), wherein the first layer (202) comprises a plurality of inlets (210) extending through the first layer (202); passing (304) the flow of air (104) through the inlets (210) into a particle collection chamber (240) between the first layer (202) and a second layer (220) of the collecting device (200) spaced apart from the first layer (202); capturing (306) airborne particles in the flow of air (104) entering the particle collection chamber (240) by impaction of airborne particles on a first surface (222) of the second layer (220), wherein ends (214) of the inlets (210) are configured to face the first surface (222) of the second layer (220); passing (308) the flow of air (104) out of the particle collection chamber (240) through outlets (230) extending through the second layer (220) of the collecting device (200); wherein the inlets (210) and outlets (230) are staggered such that the center axes of the inlets (210) are displaced from the center axes of the outlets (230); wherein the collecting device (200) is configured such that the flow of air (104) experiences a pressure drop when passing the collecting device (200), the pressure drop being lower than 3 kPa at a flow rate of 0.5 liters per second.

**Patentansprüche**

1. Sammelvorrichtung (200) zum Sammeln von Schwebeteilchen aus einem Luftstrom, wobei die Sammelvorrichtung (200) umfasst:

eine erste Schicht (202) und eine zweite Schicht (220), wobei die erste Schicht (202) und die zweite Schicht (220) dazu angeordnet sind, beabstandet zu sein, um eine Teilchensammelkammer (240) zwischen der ersten (202) und der zweiten Schicht (220) zu bilden, wobei die erste Schicht (202) eine Vielzahl von Einlässen (210) umfasst, die dazu konfiguriert sind, sich durch die erste Schicht (202) hindurch zu erstrecken, um den Luftstrom (104) durch diese hindurch in die Teilchensammelkammer (240) zu befördern;

wobei die Enden (214) der Einlässe (210) dazu konfiguriert sind, einer ersten Oberfläche (222) der zweiten Schicht (220) gegenüber zu stehen, um Schwebeteilchen in dem Luftstrom (104) aufzufangen, der durch die Enden (214) der Einlässe (210) hindurch durch Impaktion von Schwebeteilchen auf die erste Oberfläche der zweiten Schicht (220) in die Teilchensammelkammer (240) eintritt;

wobei die zweite Schicht (220) eine Vielzahl von Auslässen (230) umfasst, die dazu konfiguriert sind, sich durch die zweite Schicht (220) hindurch zu erstrecken, um den Luftstrom (104) durch diese hindurch aus der Schwebeteilchenkammer (240) heraus zu befördern;

wobei die Einlässe (210) und die Auslässe (230) gestaffelt sind, so dass die Mittelachsen der Einlässe (210) gegenüber den Mittelachsen der Auslässe (230) verlagert sind;

wobei die Sammelvorrichtung (200) derart konfiguriert ist, dass der Luftstrom einen Druckabfall erfährt, wenn er an der Sammelvorrichtung (200) vorbeigeht, wobei der Druckabfall kleiner als 3 kPa bei einem Durchfluss von 0,5 Liter pro Sekunde ist.

2. Sammelvorrichtung nach Anspruch 1, wobei ein Volumen der Teilchensammelkammer (240) kleiner als 30 μl, wie etwa kleiner als 20 μl ist.

3. Sammelvorrichtung nach Anspruch 1 oder 2, wobei die Sammelvorrichtung (200) derart konfiguriert ist, dass der Druckabfall, den der Luftstrom erfährt, wenn er an der Sammelvorrichtung (200) vorbeigeht, kleiner als 1,5 kPa bei einem Durchfluss von 0,5 Liter pro Sekunde ist.

4. Sammelvorrichtung nach einem der vorhergehenden Ansprüche, wobei die Sammelvorrichtung (200) dazu konfiguriert ist, eine Sammeleffizienz von mindestens 50 % für Teilchen aufzuweisen, die einen Durchmesser aufweisen, der größer als 300 nm ist, wenn die Sammelvorrichtung (200) einen Luftstrom (104) mit einem Durchfluss von 0,5 Liter pro Sekunde empfängt.

5. Sammelvorrichtung nach einem der vorhergehenden Ansprüche, wobei eine kleinste Dimension eines Querschnitts der Einlässe (210) in einem Bereich von 20 bis 300 μm, wie etwa in einem Bereich von 100 bis 200 μm, liegt.

6. Sammelvorrichtung nach einem der vorhergehenden Ansprüche, wobei eine Anzahl von Einlässen (210) größer als 100, wie etwa größer als 500, wie etwa 1000 bis 2000 Einlässe ist.

7. Sammelvorrichtung nach einem der vorhergehenden Ansprüche, wobei eine Länge der Einlässe (210) in einem Bereich von 20 bis 500 μm liegt, wie etwa in einem Bereich von 50 bis 300 μm, liegt.

8. Sammelvorrichtung nach einem der vorhergehenden Ansprüche, wobei ein Querschnitt der Einlässe (210) kreisförmig oder rechteckig ist.

9. Sammelvorrichtung nach einem der vorhergehenden Ansprüche, wobei eine kleinste Dimension eines Querschnitts der Auslässe (230) in einem Bereich von 20 bis 400 μm, wie etwa in einem Bereich von 100 bis 300 μm, liegt.

10. Sammelvorrichtung nach einem der vorhergehenden Ansprüche, wobei eine Anzahl von Auslässen (230) größer als 100, wie etwa größer als 500, wie etwa 1000 bis 2000 Auslässe ist.

11. Sammelvorrichtung nach einem der vorhergehenden Ansprüche, wobei eine Länge der Auslässe (230) in einem Bereich von 20 bis 500 μm, wie etwa in einem Bereich von 100 bis 300 μm, liegt.

12. Sammelvorrichtung nach einem der vorhergehenden Ansprüche, wobei die erste Schicht (202) und die zweite Schicht (220) durch eine Lücke in einem Bereich von 10 bis 150 μm, wie etwa in einem Bereich von 20 bis 100 μm, beabstandet sind.

13. Sammelvorrichtung nach einem der vorhergehenden Ansprüche, wobei eine Projektion der Einlässe (202) auf die erste Oberfläche (222) der zweiten Schicht (220) eine sechseckige Anordnung der Einlässe (210) bildet, die jeden Auslass (230) umgeben.

14. Sammelvorrichtung nach einem der vorhergehenden Ansprüche, wobei eine Projektion eines Einlasses (210) auf die erste Oberfläche (222) der zweiten Schicht (220) dazu konfiguriert ist, sich nicht mit einem nächsten benachbarten Auslass (230) zu überlappen, wie etwa eine seitliche Trennung von einer Kante der Projektion des Einlasses (210) zu einer Kante des nächsten benachbarten Auslasses (230), die mindestens 20 μm beträgt.

15. Verfahren zum Sammeln von Schwebeteilchen aus einem Luftstrom, wobei das Verfahren umfasst:

Empfangen (302) eines Luftstroms (104) auf einer ersten Schicht (202) einer Sammelvorrichtung (200), wobei die erste Schicht (202) eine Vielzahl von Einlässen (210) umfasst, die sich durch die erste Schicht (202) hindurch erstrecken;

Durchlassen (304) des Luftstroms (104) durch die Einlässe (210) in eine Teilchensammelkammer (240) zwischen der ersten Schicht (202) und einer zweiten Schicht (220) der Sammelvorrichtung (200), die von der ersten Schicht (202) beabstandet ist;

Auffangen (306) von Schwebeteilchen in dem Luftstrom (104), der durch Impaktion von Schwebeteilchen auf eine erste Oberfläche (222) der zweiten Schicht (220) in die Teilchensammelkammer (240) eintritt, wobei die Enden (214) der Einlässe (210) dazu konfiguriert sind, der ersten Oberfläche (222) der zweiten Schicht (220) gegenüberzustehen;

Durchlassen (308) des Luftstroms (104) aus der Teilchensammelkammer (240) durch Auslässe (230) hindurch, die sich durch die zweite Schicht (220) der Sammelvorrichtung (200) hindurch erstrecken;

wobei die Einlässe (210) und die Auslässe (230) gestaffelt sind, so dass die Mittelachsen der Einlässe (210) gegenüber den Mittelachsen der Auslässe (230) verlagert sind;

wobei die Sammelvorrichtung (200) derart konfiguriert ist, dass der Luftstrom (104) einen Druckabfall erfährt, wenn er an der Sammelvorrichtung (200) vorbeigeht, wobei der Druckabfall kleiner als 3 kPa bei einem Durchfluss von 0,5 Liter pro Sekunde ist.

**Revendications**

1. Dispositif de collecte (200) pour la collecte de particules en suspension dans un flux d'air, ledit dispositif de collecte (200) comprenant :

une première couche (202) et une deuxième couche (220), dans lequel la première couche (202) et la deuxième couche (220) sont espacées pour former une chambre de collecte de particules (240) entre la première (202) et la deuxième couche (220),

dans lequel la première couche (202) comprend une pluralité d'entrées (210) configurées pour s'étendre à travers la première couche (202) afin de transporter le flux d'air (104) à travers celle-ci jusqu'à la chambre de collecte de particules (240),

dans lequel les extrémités (214) des entrées (210) sont configurées pour faire face à une première surface (222) de la deuxième couche (220) afin de capturer les particules en suspension dans le flux d'air (104) entrant dans la chambre de collecte de particules (240) à travers les extrémités (214) des entrées (210) par impact des particules en suspension sur la première surface de la deuxième couche (220),

dans lequel la deuxième couche (220) comprend une pluralité de sorties (230) configurées pour s'étendre à travers la deuxième couche (220) afin de transporter l'air (104) à travers celles-ci hors de la chambre de collecte de particules (240),

dans lequel les entrées (210) et les sorties (230) sont décalées de sorte que les axes centraux des entrées (210) soient décalés par rapport aux axes centraux des sorties (230),

dans lequel le dispositif de collecte (200) est configuré de telle sorte que le flux d'air subit une chute de pression lors du passage à travers le dispositif de collecte (200), la chute de pression étant inférieure à 3 kPa à un débit de 0,5 litre par seconde.

2. Dispositif de collecte selon la revendication 1, dans lequel un volume de la chambre de collecte de particules (240) est inférieur à 30 μl, par exemple inférieur à 20 μl.

3. Dispositif de collecte selon la revendication 1 ou 2, dans lequel le dispositif de collecte (200) est configuré de telle sorte que la chute de pression subie par le flux d'air lors du passage à travers le dispositif de collecte (200) soit inférieure à 1,5 kPa à un débit de 0,5 litre par seconde.

4. Dispositif de collecte selon une quelconque des revendications précédentes, dans lequel le dispositif de collecte (200) est configuré pour fournir une efficacité de collecte d'au moins 50 % pour les particules d'un diamètre supérieur à 300 nm lorsque le dispositif de collecte (200) reçoit un flux d'air (104) à un débit de 0,5 litre par seconde.

5. Dispositif de collecte selon une quelconque des revendications précédentes, dans lequel une dimension la plus petite d'une section transversale des entrées (210) est comprise entre 20 et 300 μm, par exemple entre 100 et 200 μm.

6. Dispositif de collecte selon une quelconque des revendications précédentes, dans lequel le nombre d'entrées (210) est supérieur à 100, par exemple supérieur à 500, par exemple entre 1 000 et 2 000.

7. Dispositif de collecte selon une quelconque des revendications précédentes, dans lequel la longueur des entrées (210) est comprise entre 20 et 500 μm, par exemple entre 50 et 300 μm.

8. Dispositif de collecte selon une quelconque des revendications précédentes, dans lequel la section transversale des entrées (210) est circulaire ou rectangulaire.

9. Dispositif de collecte selon une quelconque des revendications précédentes, dans lequel une dimension la plus petite d'une section transversale des sorties (230) est comprise entre 20 et 400 μm, par exemple entre 100 et 300 μm.

10. Dispositif de collecte selon une quelconque des revendications précédentes, dans lequel le nombre de sorties (230) est supérieur à 100, par exemple supérieur à 500, par exemple entre 1000 et 2000 sorties.

11. Dispositif de collecte selon une quelconque des revendications précédentes, dans lequel la longueur des sorties (230) est comprise entre 20 et 500 μm, par exemple entre 100 et 300 μm.

12. Dispositif de collecte selon une quelconque des revendications précédentes, dans lequel la première couche (202) et la deuxième couche (220) sont espacées d'un espace compris entre 10 et 150 μm, par exemple entre 20 et 100 μm.

13. Dispositif de collecte selon une quelconque des revendications précédentes, dans lequel une projection des entrées (202) sur la première surface (222) de la deuxième couche (220) forme un agencement hexagonal des entrées (210) entourant chaque sortie (230).

14. Dispositif de collecte selon une quelconque des revendications précédentes, dans lequel une projection d'une entrée (210) sur la première surface (222) de la deuxième couche (220) est configurée pour ne pas se superposer à une sortie (230) la plus proche, par exemple, de sorte qu'un écart latéral entre un bord de la projection de l'entrée (210) et un bord de la sortie (230) la plus proche soit d'au moins 20 μm.

15. Procédé de collecte de particules en suspension dans l'air dans un flux d'air, ledit procédé comprenant :

la réception (302) d'un flux d'air (104) sur une première couche (202) d'un dispositif de collecte (200), dans lequel la première couche (202) comprend une pluralité d'entrées (210) s'étendant à travers la première couche (202) ;
le passage (304) du flux d'air (104) à travers les entrées (210) dans une chambre de collecte de particules (240) entre la première couche (202) et une deuxième couche (220) du dispositif de collecte (200) espacé de la première couche (202) ;
la capture (306) des particules en suspension dans le flux d'air (104) entrant dans la chambre de collecte de particules (240) par impact des particules en suspension sur une première surface (222) de la deuxième couche (220), dans lequel les extrémités (214) des entrées (210) sont configurées pour faire face à la première surface (222) de la deuxième couche (220) ;
l'évacuation (308) du flux d'air (104) hors de la chambre de collecte de particules (240) par des sorties (230) s'étendant à travers la deuxième couche (220) du dispositif de collecte (200) ;
dans lequel les entrées (210) et les sorties (230) sont décalées de sorte que les axes centraux des entrées (210) soient décalés par rapport aux axes centraux des sorties (230) ;
dans lequel le dispositif de collecte (200) est configuré de telle sorte que le flux d'air (104) subisse une chute de pression lors du passage à travers le dispositif de collecte (200), la chute de pression étant inférieure à 3 kPa à un débit de 0,5 litre par seconde.

Fig. 1

Fig. 2

EP 4 229 390 B1

Fig. 3

Fig. 4

210    230

*Fig. 5*

210    240    200

150μm    100μm    202

20μm    30μm

320μm    220

150μm

230

*Fig. 6*

*Fig. 7*

*Fig. 8*

RECEIVE
FLOW OF AIR ONTO
A PLURALITY OF INLETS
OF COLLECTING
DEVICE

— 302

PASS FLOW
OF AIR THROUGH
THE INLETS INTO A
PARTICLE COLLECTION
CHAMBER

— 304

CAPTURE AIRBORNE
PARTICLES IN THE
PARTICLE COLLECTION
CHAMBER BY IMPACTION
ON A FIRST SURFACE OF
SECOND LAYER OF
COLLECTING DEVICE

— 306

PASS FLOW OF
AIR OUT OF PARTICLE
COLLECTION CHAMBER
THROUGH OUTLETS
EXTENDING THROUGH
SECOND LAYER

— 308

*Fig. 9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 20201787
- EP 21170431 A
- US 20160223435 A1
- US 20200278275 A1

**Non-patent literature cited in the description**

- **MARPLE** ; **WILLEKE**. Impactor Design. *Atmospheric Environment*, 1976, vol. 10 (10), 891-896